# EUROPEAN PATENT APPLICATION

(11) **EP 2 194 480 A1**
(43) Date of publication of application: **09.06.2010**
(21) Application number: 09163180.4
(22) Date of filing: 18.06.2009
(51) Int. Cl.: G06F 21/00

(54) **Device for management of personal data**

(30) Priority: 05.12.2008 US 329191
(71) Applicant: NCR Corporation, Duluth, GA 30096 (US)
(72) Inventor: Kobres, Erick, Lawrenceville, GA 30043 (US)
(74) Representative: MacLeod, Roderick William

(57) **Abstract**

A portable device (3) which stores and transmits personal information relating to a person, to simplify transfer of such information in transactions. The device (3) contains a first database (6), such as a medical history of a person, which is encrypted. Specific parties, such as medical personnel, are authorized to gain access to the first database, but not other parties. The device (3) de-crypts the first database (6) and transmits the data to the authorized parties. Similar procedures are undertaken with respect to other databases (6) contained within the device.

## Description

The invention concerns a portable device which stores personal information of its owner, and transfers selected information to selected parties in connection with transactions undertaken by the owner.

People interact with other people and institutions, and divulge information about themselves on a continuing basis. Some of the information is non-confidential, and is freely disclosed. For example, a person visiting a hair stylist will express a preference as to how his/her hair should be done. As another example, a person making a reservation for an airline ticket may have preferences as to seating and type of food.

In contrast, other information is considered confidential, and is not freely disclosed. Confidential information would include financial information, tax returns, medical information, and so on.

Apart from confidentiality issues in information, people also disclose the same information repeatedly. For example, when a person orders merchandise over the Internet, the person provides his name, address, telephone number, and credit card number each time an order is placed.

The invention provides an improved system for storing personal information and for selectively transmitting the information to third parties.

An object of an embodiment of the invention is to provide an improved management system for personal information.

A further object of an embodiment of the invention is to provide an improved management system for personal information which provides access to different types of information to different third parties.

In one form of the invention, medical information about a person is encrypted and stored in a portable device. Authorized medical personnel are granted access to the information, but other parties are denied access.

According to a first aspect of the invention there is provided a portable device as set forth in claim 1.

According to a second aspect of the invention there is provided a method as set forth in claim 6.

According to a third aspect of the invention there is provided a system as set forth in claim 7.

According to a fourth aspect of the invention there is provided a portable device, comprising:
a) an access control system which
   i) grants access to a database A to a party A;
   ii) grants access to a database B to a party B;
   iii) denies access to database A to party B; and
   iv) denies access to database B to party A; and
b) an input control system which examines data submitted by party A and, if the submitted data meets predetermined criteria, stores the submitted data.

Database A may be located remotely from the portable device.

Party A may be a merchant and database A may contain financial account data which allows an owner of the portable device to make a purchase from party A.

The submitted data may comprise a reward given in response to said purchase.
Figure 1 illustrates one embodiment of the invention.
Figure 2 illustrates architecture implemented by one embodiment of the invention.

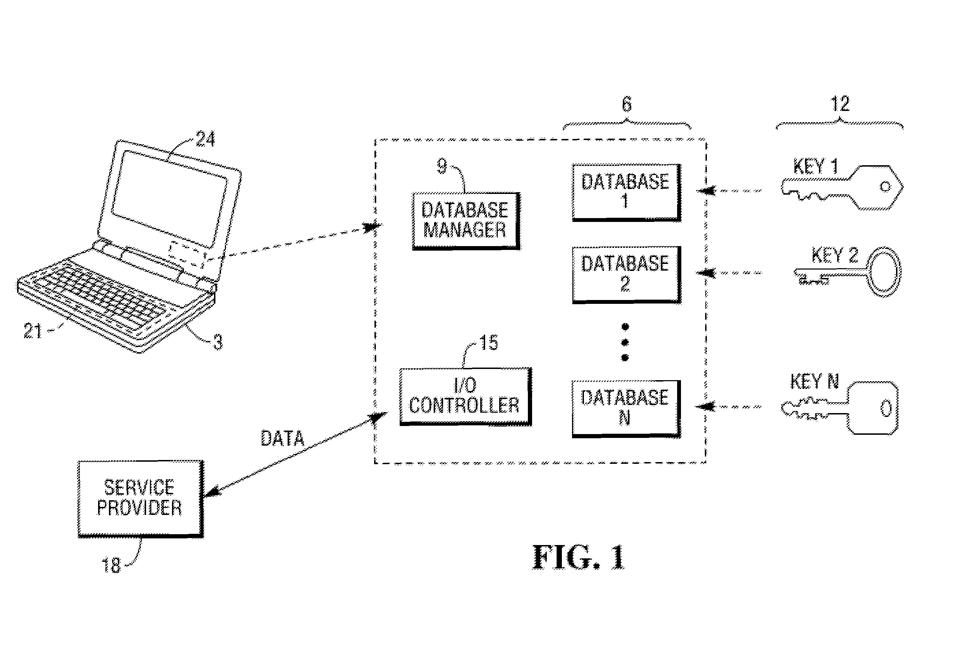

Figure 1 illustrates a portable device 3 which is carried by a person. This device 3 generically represents a small computer, and can be implemented by numerous commercially available products, such as Personal Digital Assistants (PDAs), cell phones, Blackberries (TM), and so on.

The device 3 contains multiple databases 6, a number N of which are indicated. Each database 6 contains a different class, or type, of information. For example, one database may contain medical records. Another database may contain tax returns. A third database may contain credit card information, such as information required to make credit card purchases. The third database may also contain additional information required to make a purchase over the telephone, such as a shipping address.

A fourth database may contain photographs of the person which are used for various purposes, such as identification or indicating to a barber how the person wishes a haircut to be performed.

A fifth class of databases, which could be numbered databases 100 through 200, may contain generic, non-confidential information about the person, such as (1) the type of music preferred (classical, rock-and-roll, musical theater, etc.), (2) preferences in video entertainment, (3) favorite colors in clothing, and so on.

The databases 6 are stored in encrypted form, and encryption and de-cryption is handled by a database manager 9. It is possible that the non-confidential databases are not encrypted.

An encryption key 12 is required to de-crypt each database, although it is possible that a single key 12 may de-crypt more than one database. The same key may be used for encryption of data as it is placed into a database, or a different key may be used, depending on the preferences of the designer.

An input-output (I/O) controller 15 transfers data to and from third parties, one of which is represented by a service provider 18.

In one mode of operation, the person owning the device 3 visits a medical clinic, and the device 3 carries the person's medical history in the form of encrypted database 1. Personnel in the clinic enter key 1 into the device 3, which causes the database manager 9 to de-crypt database 1, and transmit database 1 to the clinic.

Key 1 can be entered into the device 3 in any number of ways. For example, it can be punched into a keypad 21 within the device. However, since encryption keys tend to be large numbers, such keypunching is not preferred. As another example, the key can be entered using wireless data transfer technologies, such as that known as Bluetooth (TM). As another example, the key can be swiped in, using a card resembling a credit card, a smart card, a USB key-fob memory stick, or the like.

In one form of the invention, only the plain text of the database 1 is transferred to the clinic. That is, the encrypted version of database 1 is kept within the device 3, and is not transferred. One reason for this restriction is that known cryptographic principles state that both the plain text and cypher text of a message should never be given to a third party. Such access provides the third party with an advantage in deducing (1) the type of algorithm used to encrypt the plain text, (2) the type of key used, or (3) both (1) and (2).

In another mode of operation, the owner of device 3 visits a hair salon. The owner locates database 2, which contains a photograph of the hair style which the person prefers, and presents the photograph to the stylist. Since such a photograph is probably not considered confidential, the photograph is not encrypted, and is accessible directly through selection of a menu (not shown) on a display 24 of the device 3.

The person may wish to pay the hair stylist using a credit card, the data of which is encrypted and stored in database 3. The person arranges for the key to database 4 to be entered into the device 3, which causes the de-crypted credit card number, and other required information, to be transferred to a POS, point of sale terminal, at the salon, which is represented by service provider 18.

Payment to the medical clinic can be made in the same manner.

In another mode of operation, the owner of device 3 may wish to purchase a book over the Internet. The person uses a computer to find the web site of a book merchant (or the person may use device 3 for this purpose). When the purchase is to be made, the person enters the appropriate key 12 for the database which contains encrypted credit card information. The device 3 then transmits the credit card information to the computer which is connected to the Internet, or transmits the information itself to the web site, if the device 3 is being used to browse the Internet.

Therefore, as so far explained, the device 3 contains multiple databases 6. A subset of these databases 6 is encrypted. Another subset is not, although, in one form of the invention, all databases can be encrypted. Each encrypted database requires a different key for de-cryption, although it is possible that a single key de-crypts several of the databases.

Some details of implementation of this embodiment of the invention will now be considered.

A published standard which defines the layout, or schema, of each database will be generated, so that parties such as the medical clinic discussed above, which wish to gain access to the databases, can do so easily by compliance with the published standard.

For example, a set of different types of databases will be defined by an organization. The types of databases may include (1) medical histories, (2) educational transcripts, (3) credit card purchasing information, (4) automobile repair records, (5) tax returns, and so on.

For each type of database, the standardized approach also defines the format, or schema, of that database. This allows users of the database to more easily search the database. For example, if the database is a telephone directory, the format can be defined as (1) family name, (2) given name, (3) street name, (4) house number on street, (5) city name, (6) state name, and (7) postal code. In addition, the standard states that entries are stored in alphabetical order according to family name. Thus, if the user is looking for the family name "Zieman," he need not make a brute-force search of the entire database, beginning with the "A's", but can proceed in a more orderly manner.

Of course, if the person is looking for a specific telephone number, the knowledge that the telephone directory is arranged alphabetically by family name is not necessarily helpful. In this case, and in general as well, various indexing schemes, as known in the science of database management, can be implemented. Thus, the representation of databases 6 in Figure 1 also is a representation of associated indexing of the databases.

It is possible that manufacturers of the devices 3 will be the parties who are most interested in establishing the standards just described. It is also possible that these manufacturers may not agree on standards to be defined. Therefore, for a given type of database, such as a medical history, each manufacturer may define its own standard. The database within the device 3 will contain a notation indicating the specific standard to which it conforms.

For example, each database 6 may contain (1) a descriptive title, such as "medical history," and (2) a statement identifying the format or schema by which the database is organized, and possibly (3) identification of a web site on the Internet which contains the identifying format or schema for the database. In this manner, while the medical history is not necessarily organized according to one fixed schema, nevertheless, it is organized according to one of a few possible schemas. Those possible schemas are publicly available to the user of the database.

The system can be implemented using common encryption, digital certificate and verification standards generally available today, and extended to future technologies as necessary. The system can be implemented upon any number of platforms capable of storing information and performing the calculations necessary to encrypt, decrypt, digitally sign, and verify the authenticity of signed information. Significant additional value can be realized through the optional inclusion of a mechanism capable of wirelessly transmitting and receiving information.

The system employs a number of significant concepts, including the following.
1. The system maintains encrypted databases of facts.
2. The system maintains an encrypted database of data management policies, which control which parties are to be granted access to the databases.
3. The system maintains an encrypted database of public and private keys or certificates associated with the producers and consumers of facts. The database of keys may include the key or keys used to encrypt the other databases.
4. The system maintains an encrypted database of fact classes which define data structure, policy rules and other metadata about facts that can be stored in the system.
5. The system employs a policy engine which coordinates use of the data in the fact, key, class and policy databases to provide the services of storing, managing and retrieving facts.
6. All information, or selected information, stored by the system is digitally signed by the owner of the information, and encrypted with a private key, or with authentication credentials based on well-protected criteria such as locations, webs of trust, biometric information, strong passwords, token possession, or a combination of these or similar mechanisms.
7. All information, or selected information, disclosed by the system is digitally signed with the public key of the recipient of the information.
8. Information disclosed by the system may be recorded in a transaction log such that the public key, time, date, and what were disclosed are recorded and encrypted.
9. The user of the system (and the owner of the information stored therein) controls whether information is disclosed to an entity seeking information.
10. Classes of information are defined in a public registry. For example, an address as a class of information will have a pre-defined schema, as will a name, a contact, a calendar entry, a task item, as will a restaurant seating preference, as will any kind of information expected to be stored in such a system.
11. Classes of information are defined with a default or recommended privacy level. For example, a person's medical history would be classified by default at a very high level of security while his or her seating preference may be classified by default as public information.
12. Classes of information consumers are defined in a public registry, which is generated by a third party, who is different from the owner of the portable device. For example, retailers, emergency personnel and government agencies, medical establishments, individuals, airlines, financial institutions and so forth.
13. Class groups are defined in a public registry, which is generated by a party other than the owner of the device 3, and can be specified to include all of a particular authenticated class of information or information consumer. For example, a user can specify that they wish to disclose all information of the category "medical emergency information" to anyone with the categorization of "emergency medical personnel" while specifying that "detailed personal medical information" cannot be disclosed to anyone without express authorization.
14. Preferences as to how information may be disclosed by the system can be controlled by the user of the system. For example, one user may desire to approve all disclosure by secure authentication while another user may elect to make certain information openly and freely available.
15. The system storing the repository advertises and/or responds to solicitations from authorized fact consumers and producers wirelessly.
16. The system can utilize information from location awareness technologies such as GPS, wireless triangulation and well-known hotspots.

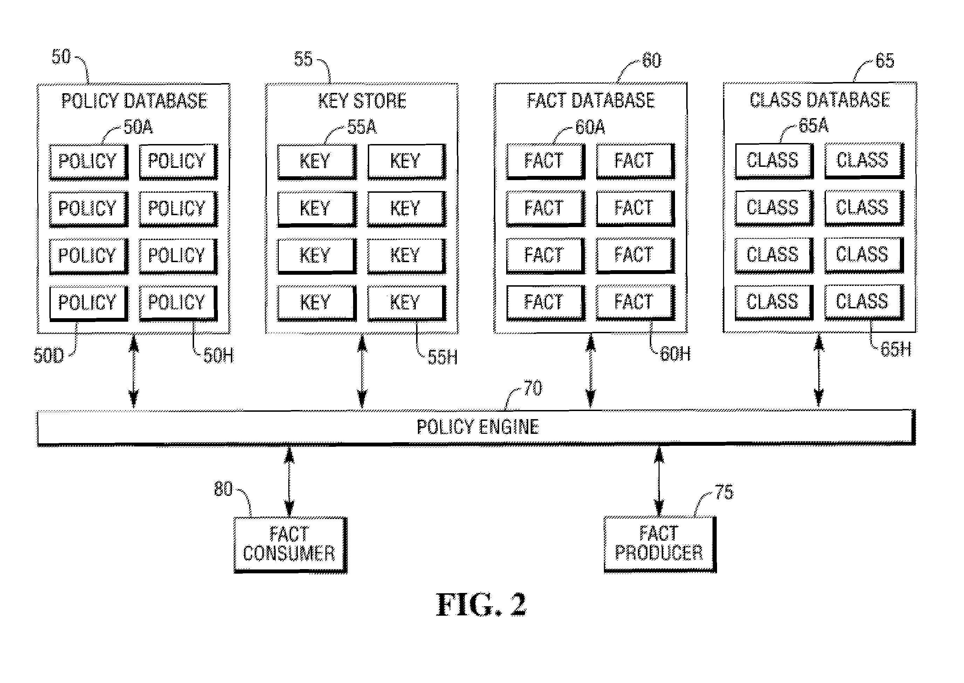

Figure 2 represents an architecture used by one form of the invention. Block 50 represents a policy database, with policies 50A - 50H contained therein. The policies define the restrictions placed on disclosure of the contents of the databases.

Block 55 represents a key storage unit, which stores the encryption keys 55A - 55H for the respective databases.

Block 60 represents a database of facts, and represents the contents of eight databases 60A - 60H.

Block 65 represents a class database, and represents classes 65A - 65H. The classes define the parties who are entitled to gain access to the databases and also, optionally, whether a party is only entitled to a specific subset of a database and, if so, the identity of the subset.

Block 70 represents a policy engine, which handles transmissions into, and out of, the databases, between fact producers 75 (e.g., the owner of the device 3 in Figure 1), and fact consumers 80 (e.g., the medical clinic discussed above).

### Significant Features

1. Some data stored within the portable device 3 is considered more sensitive, or more private, than other data. For example, a medical history is considered more sensitive than a seating preference in an aircraft. In one form of the invention, the more sensitive data is encrypted using a more secure algorithm than the less sensitive data. One of the features of a more secure algorithm is that, using a given computer, the processing time required for encryption and decryption is greater than for a less secure algorithm. Another feature can be that the key length for the more secure algorithm is longer than for the less secure algorithm.
2. If data is not encrypted, then the key length is defined as zero, and the processing time for a de-cryption algorithm is also defined as zero. Thus, data which is weakly encrypted, or not encrypted at all, may have a shorter key length than data which is strongly encrypted, and also has an algorithm which is less secure than the algorithm used for the more strongly encrypted data.
3. Some basic concepts of organizing a database are used by the invention. An ordinary telephone directory is a type of database, as explained above. By convention, the position of an item in each entry (an "entry" is one line in the "white pages") indicates the identity of each item, or defines the meaning of that item.
   For example, the items "Jackson Jerry" indicate that "Jackson" is the family name and "Jerry" is the given name. The person's name is "Jerry Jackson," and not "Jackson Jerry." Accordingly, for each entry, or line, in a United States telephone directory, items 1, 2, 3, 4, 5, 6, 7, and 8, in that order, correspond, respectively, to
   family name,
   given name,
   street number,
   street name,
   city name,
   state name,
   zip code, and
   telephone number.
   This illustrates the principle that a convention can be set up in which relative position within a database can indicate the meaning of an item at a given position. In the example above, the number in the seventh position is a zip code. The individual items are not labeled, but are defined, according to a convention, by their position in the entry, that is, by their position in the line of data.
   In a database which represents a medical history of a person, position can be used in a similar way. For example, the database may contain 1,000 items. Items 450 through 499 can be assigned to medical treatments received from ages 10 through 12, and so on.
   In another approach, position is not used to define each item. Instead, each item in the database is labeled and, in effect, is treated as a character string. In the telephone directory example given above, the labeling may be "Family name = Jackson," "Given name = Jerry," and so on. Of course, the labels increase the size of the database, and are not used in a simple database such as a telephone directory. Under this approach, database management software searches the database for the label desired, to find the information desired.
   Other approaches are possible.
   The particular mode of organizing the database is often called a "schema," or the format of the database. Knowledge of the schema, or format, allows a person to find information within the database. If the schema is not known, then finding desired information may be extremely difficult.
   Schema is a term of art, and is defined in the science of database design.
4. In one form of the invention, an encryption key acts as identification of a party seeking access to a database within the portable device 3. Thus, merely presenting the key causes the device 3 to de-crypt the corresponding database, and transmit the plain text of the database.
   In another form of the invention, independent identification of a party seeking access is required. The database manager 9 or the I/O controller 15, or both, assess the identification presented and, if it meets specified criteria, then accept an encryption key to allow the decryption.
   In yet another form of an embodiment of the invention, identification alone of a consumer of data may be sufficient. That is, a party submits sufficient information to identify himself. After identification is successful, the invention identifies the class of database(s) to which the party has access, locates the corresponding key, and delivers plain text of the data.
5. In one form of an embodiment of the invention, the encryption keys are stored within the device 3. The keys can themselves be encrypted. A person seeking access presents proper identification, as described above. If the person is authenticated, the device 3 retrieves the key, de-crypts it if necessary, and de-crypts the corresponding database. The person may be required to submit a key which de-crypts the necessary stored keys.
6. A public registry, generated by a person other than the owner of the device 3, defines classes of consumers of data, such as retail merchants, emergency room medical personnel, and so on. The registry also specifies the types of databases within the device 3 to which each class of consumers is granted access. When a member of a class presents proper identification, access is granted to the corresponding classes.
   The owner of the device 3 is granted authority to modify these definitions.
   A class will contain more than two entities, and does not refer to a specific individual. For example, the class of emergency room medical personnel refers to all emergency rooms in all hospitals, or a group of hospitals defined by the user of the device 3.
   If a single entity, such as a specific emergency room in a specific hospital, is intended, then that specific entity is identified, and the class is then termed a "limited class." Since this limited class contains only one member, it is not defined as a "class."
7. A third party can define the format, or schema, of data within the portable devices. Two or more devices, owned by two different people, can be in existence, and both will contain data within them which will be arranged according to the same schema, although the specific data, in general, will be different. For example, both devices may contain medical information, which is organized according to the same schema, but, of course, the information will be different in the two devices, since the two people are different.
   To repeat: different devices can contain databases which are defined within a given class (e.g., medical history), those databases will be organized according to the same schema, but their contents will be different.
8. Some data stored within the device 3 can be defined as "non-sensitive." Such data would include that which can be obtained by lawful observation of a person while the person is in a public place. For example, a person's choice of seating in a restaurant, or choice of seating on an aircraft, or choice of a make and model of automobile, are all observable in this manner, and are non-sensitive.
   In contrast, a person's tax returns, credit card statements, and medical history are not so observable.
9. The device 3 acts as an interface; it need not store the data to be transferred. For example, some or all of the data indicated in the Figures can be stored at a web site, or remote computer, such as the person's home computer equipped with a modem or network access. The device 3 allows a data consumer to gain access to the stored data as described above.
10. While the invention extends to a device, a very similar approach applies to a web site, email account or other computer system which stores the databases indicated in the Figures. In one form of the invention, a computer would almost certainly be needed as part of the system to enroll and manage most data. Doing so on a device would be possible, but cumbersome.
11. The invention provides for the ability to selectively receive information from third parties using the same type of policies.
12. Implementation of one form of the invention does not require the device 3. A person could carry the database on a storage media, access it through an online portal, or access a copy of it stored on the portable device.
13. In connection with one of the comments above, it is pointed out that disclosure may be allowed to be automatic based on policy settings.
14. In some situations, there may even be an enforced level of security, which is beyond control of the owner of the device, for certain classes such as for bank card information.
15. The system can accomplish its goals over any other communication channel. For example the database could be used in this way as a spam filter such that only signed advertisements passing the policy rules are allowed into the inbox.
16. It is possible that the information can be transmitted encrypted with the public key or some form of derived unique key of the recipient of the information so that "transmission in the clear" is not required.
17. In another form of the invention, email clients, social networking sites and other potential target platforms can enable use of the database by a consumer of the data.
18. In another form of the invention, the consumer receives information, as above. In addition, the same architecture and communication with the consumer is used, but the consumer becomes the publisher, and a policy determines whether the user (i.e., the owner of the device) will accept the information.

For example, if the user makes a purchase, the user receives a loyalty reward, and electronic receipt, and perhaps some other offer from a retailer, all of which are stored in the device. This is significant because it is the channel through which retailer and institutional value is created, enabling interested business to subsidize the cost of the implementation and make it available for free to the end user.

Numerous substitutions and modifications can be undertaken without departing from the true spirit and scope of the invention.

## Claims

1. A portable device (3), comprising:
a) a first collection of data which
i) is encrypted, and
ii) requires a first key (12) for de-cryption;
b) a second collection of data which is not encrypted;
c) a set of access definitions (50), which identify parties who are authorized to gain access to the first collection of data;
d) a control system (9) which
i) identifies a party A seeking data from the first collection of data, and
ii) determines whether the access definitions authorize the party A to gain access to the first collection of data and, if so, de-crypts data within the first collection and transmits resulting plain text to party A.

2. A device as in claim 1, in which the control system (9) transmits data from the second collection to a party B seeking access, without identifying party B.

3. A device as in claim 1, and further comprising:
e) a third collection of data which
i) is encrypted, and
ii) requires a third key (12) for de-cryption; and
f) in which the control system (9) grants access to the third collection to a party C, but not to party B.

4. A device as in claim 1, in which the first collection of data comprises a medical history of a person owning the device.

5. A device as in claim 2, in which the second collection of data comprises information which is obtainable by lawful observation of a person owning the device while in a public place.

6. A method of operating a portable device (3), comprising:
a) maintaining within the device (3) first data which indicates medical history of a person, said data being encrypted and requiring a first key (12) for decryption;
b) presenting the device (3) to a medical services provider;
c) receiving identifying data from the provider, and, if the identifying data meets predetermined criteria, using said key (12) to de-crypt data, and transmitting resulting plain text to the provider;
d) maintaining within the device (3) second data which indicates a payor for services;
e) presenting the device (3) to a billing agent of the provider; and
f) receiving identifying data from the billing agent, and, if the identifying data meets predetermined criteria, instructing the payor to submit a payment to the billing agent.

7. A system, comprising:
a) a portable device A, which contains
i) a database A which
A) comprises a medical history of a person A,
B) is encrypted using a key A, and
C) conforms to a schema A; and
ii) a database B which is not encrypted;
b) a portable device C, which contains
i) a database C which
A) comprises a medical history of a person C,
B) is encrypted using a key C, and
C) conforms to said schema A; and
ii) a database D which is not encrypted;
c) a registry, accessible to devices A and C, which defines
i) parties A who are granted access to database A, and
ii) parties C who are granted access to database C;
d) a control system in device A which restricts access to database A to parties A; and
e) a control system in device C which restricts access to database A to parties C; and
f) a publicly available definition of schema A.
